# EUROPÄISCHE PATENTSCHRIFT

(11) **EP 2 673 310 B1**
(45) Veröffentlichungstag und Bekanntmachung des Hinweises auf die Patenterteilung: **19.11.2014**
(21) Anmeldenummer: 12702040.2
(22) Anmeldetag: 06.02.2012
(51) Int. Cl.: C08G 18/10, C08G 18/38, C08G 18/42, C08G 18/73, A61L 15/26

(54) **GEWEBEKLEBER AUF BASIS STICKSTOFFMODIFIZIERTER ASPARTATE**
TISSUE ADHESIVE BASED ON NITROGEN-MODIFIED ASPARTATES
ADHÉSIF TISSULAIRE À BASE D'ASPARTATES MODIFIÉS PAR AZOTE

(30) Priorität: 09.02.2011 EP 11153810
(43) Veröffentlichungstag der Anmeldung: 18.12.2013
(73) Patentinhaber: Medical Adhesive Revolution GmbH, 52074 Aachen (DE)
(72) Erfinder: HECKROTH, Heike, 51519 Odenthal (DE); EGGERT, Christoph, 50733 Köln (DE)
(74) Vertreter: Davepon, Björn
(86) Internationale Anmeldenummer: PCT/EP2012/051914
(87) Internationale Veröffentlichungsnummer: WO 2012/107375

(56) Entgegenhaltungen:
- EP-A1- 2 083 025
- EP-A1- 2 275 466
- EP-B1- 2 097 466
- WO-A2-2008/076707

## Beschreibung

Die vorliegende Erfindung betrifft ein Polyharnstoff-System insbesondere zum Verschließen, Verbinden, Verkleben oder Abdecken von Zellgewebe sowie ein Dosiersystem für das erfindungsgemäße Polyharnstoff-System.

Diverse Materialien, die als Gewebekleber eingesetzt werden, sind im Handel erhältlich. Hierzu gehören die Cyanacrylate Dermabond^{®} (Octyl-2-Cyanoacrylat) und Histoacryl Blue^{®} (Butyl-Cyanoacrylat). Voraussetzung für eine effiziente Klebung der Cyanacrylate sind allerdings trockene Untergründe. Bei starken Blutungen versagen derartige Klebstoffe.

Als Alternative zu den Cyanacrylaten stehen biologische Klebstoffe wie zum Beispiel BioGlue^{®}, eine Mischung aus Glutaraldehyd und Bovinem Serumalbumin, diverse kollagen- und gelatinebasierte Systeme (FloSeal^{®}) sowie die Fibrinkleber (Tissucol) zur Verfügung. Diese Systeme dienen in erster Linie der Blutstillung (Hämostase). Neben den hohen Kosten zeichnen sich Fibrinkleber durch eine relative schwache Klebestärke und einen schnellen Abbau aus, so dass sie nur bei kleineren Verletzungen auf nicht gespanntem Gewebe verwendet werden können. Kollagen- und Gelatinebasierte Systeme wie FloSeal^{®} dienen ausschließlich der Hämostase. Zudem besteht, da Fibrin und Thrombin aus humanem-, Collagen und Gelatine aus tierischem Material gewonnen werden, bei biologischen Systemen immer die Gefahr einer Infektion. Biologische Materialien müssen außerdem gekühlt gelagert werden, so dass ein Einsatz in der Notfallversorgung wie z. B. in Katastrophengebieten, bei militärischen Einsetzen etc. nicht möglich ist. Hier steht zur Behandlung traumatischer Wunden QuikClot^{®} oder QuikClot ACS+™ zur Verfügung, welches ein mineralisches Granulat ist, das im Notfall in die Wunde gebracht wird und dort durch Wasserentzug zur Koagulation führt. Im Falle von QuikClot® ist dies eine stark exotherme Reaktion, die zu Verbrennungen führt. QuikClot ACS+™ ist eine Gaze, in die das Salz eingebettet ist. Das System muss zur Blutstillung fest auf die Wunde gedrückt werden.

Aus der WO 2009/106245 A2 ist die Herstellung und Verwendung von Polyharnstoff-Systemen als Gewebekleber bekannt. Die hier offenbarten Systeme umfassen wenigstens zwei Komponenten. Dabei handelt es sich um einen aminofunktionellen Asparaginsäureester und ein isocyanatfunktionelles Prepolymer, das durch Umsetzung von aliphatischen Polyisocyanaten mit Polyesterpolyolen erhältlich ist. Die beschriebenen 2-Komponenten Polyharnstoff-Systeme können als Gewebekleber für den Verschluss von Wunden in menschlichen und tierischen Zellverbänden eingesetzt werden. Dabei kann ein sehr gutes Klebeergebnis erzielt werden.

Aus WO 2008/076707 ist die Herstellung von sekundären Aminogruppen enthaltende Aminofunktionellen Asparaginsäureestem bekannt. Zur Herstellung der Asparagineester wird Bishexamethylen-Triamin eingesetzt und die daraus hergestellte Asaparagineester werden mit einem Prepolymer hergestellt aus Polytetramethylenglykol (MG 650) und Isophorondiisocyanat umgesetzt. Die hieraus hergestellte Polyharnstoffe werden als Beschichtungmittel eingesetzt.

In EP 2 097 466 werden ebenfalls sekundäre Aminogruppen enthaltende aminofunktionelle Asparagineester offenbart zur Verwendung in Beschichtungsmitteln.

Um eine gute Mischbarkeit der beiden Komponenten des Polyharnstoff-Systems sicher zu stellen, sollte die Viskosität der Komponenten bei 23 °C möglichst kleiner als 10.000 mPas sein. Eine entsprechend niedrige Viskosität weisen Prepolymere mit NCO-Funktionalitäten von weniger als 3 auf. Wenn derartige Prepolymere eingesetzt werden, ist es notwendig, als zweite Komponente einen Asparaginsäureester mit einer Aminofunktionalität von mehr als 2 einzusetzen, da ansonsten kein polymeres Netzwerk hergestellt werden kann. Dies ist jedoch erforderlich, damit das Polyharnstoff-System bzw. eine daraus bestehende Klebnaht die gewünschten mechanischen Eigenschaften wie Elastizität und Festigkeit aufweist. Darüber hinaus ist bei der Verwendung difunktioneller Asparaginsäureester nachteilig, dass die Aushärtungszeit bis zu 24 h beträgt, wobei das Polyharnstoff-System selbst nach dieser Zeit in vielen Fällen klebrig bleibt, also nicht tack free ist.

Aufgabe der Erfindung war es daher, ein Polyharnstoff-System bereit zu stellen, das eine gute Mischbarkeit aufweist und schnell unter Ausbildung eines dreidimensionalen Polyharnstoff-Netzwerks ausreagiert. Dabei war als zusätzliche Bedingung zu beachten, dass das ausgehärtete System nach ISO 10993 bei der Anwendung im Menschen oder im Tier keine Zytotoxizität aufweist.

Dieses Aufgabe ist erfindungsgemäß durch ein Polyharnstoff-System, umfassend
als Komponente A) isocyanatfunktionelle Prepolymere erhältlich durch Umsetzung von aliphatischen Isocyanaten A1) mit
   Polyolen A2), die insbesondere ein zahlenmittleres Molekulargewicht von ≥ 400 g/mol und eine mittlere OH-Funktionalität von 2 bis 6 aufweisen können,
als Komponente B) aminofunktionelle Asparaginsäureester der allgemeinen Formel (I) 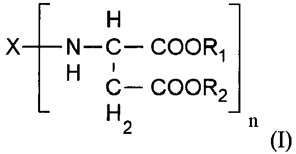 in der
   - X: ein organischer Rest enthaltend eine sekundäre Aminofunktion ist,
   - R₁, R₂: gleiche oder verschiedene organische Reste sind, die keinen Zerewitinoffaktiven Wasserstoff aufweisen und
   - n: eine ganze Zahl von mindestens 2 ist,
gelöst.

Die Komponenten des erfindungsgemäßen Polyharnstoff-Systems können leicht miteinander vermischt werden, da sie bei 23 °C eine Viskosität von weniger als 10.000 mPas aufweisen. Darüber hinaus können sie nach dem Vermischen schnell ein dreidimensionales Polyharnstoff-Netzwerk bilden. Dieses Netzwerk zeichnet sich durch hohe Elastizität, Festigkeit, Klebstärke und einen Fehlen von Zytotoxizität aus. Außerdem ist das Netzwerk bereits nach kurzer Zeit nicht mehr klebrig, d.h. tack free.

Besonders bevorzugt ist n in der Formel (I) eine ganze Zahl ≥2 ≤ 4 und ganz besonders bevorzugt gleich 2.

Gemäß einer weiteren bevorzugten Ausführungsform des erfindungsgemäßen Polyharnstoff-Systems ist X ein Rest der Formel (II) 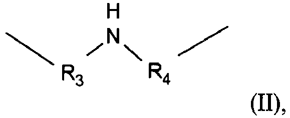 in der
- R₃, R₄: jeweils unabhängig voneinander ein organischer Rest sind, der keinen Zerewitinoffaktiven Wasserstoff aufweist.

Besonders bevorzugt ist hier, wenn R3, R4 jeweils unabhängig voneinander oder gleichzeitig ein linearer oder verzweigter, gegebenenfalls auch in der Kette mit Heteroatomen substituierter gesättigter organischer Rest, insbesondere ein linearer oder verzweigter, gesättigter, aliphatischer C1 bis C10, bevorzugt C2 bis C8 und ganz besonders bevorzugt C2 bis C6 Kohlenwasserstoffrest sind. Derartige Polyharnstoff-Systeme härten besonders schnell aus.

Ebenfalls vorteilhaft ist ein Polyharnstoff-System umfassend eine Verbindung der Formel (I) in der die Reste R₁, R₂ jeweils unabhängig voneinander lineare oder verzweigte C1 bis C10, bevorzugt C1 bis C8, besonders bevorzugt C2 bis C6, ganz besonders bevorzugt C2 bis C4 organische Reste und insbesondere aliphatische Kohlenwasserstoffreste sind. Beispiele besonders geeigneter Reste sind Methyl, Ethyl, Propyl und Butyl.

Die erfindungsgemäßen Polyharnstoff-Systeme werden durch Mischung der Prepolymere A) mit den aminofunktionellen Verbindung B) sowie gegebenenfalls den Komponenten C), D) und/oder E) erhalten. Das Verhältnis von freien oder blockierten Aminogruppen zu freien NCO-Gruppen beträgt dabei bevorzugt 1:1,5, besonders bevorzugt 1:1. Wasser und/oder Amin werden dabei der Komponente B) bzw. C b) beigemischt.

Die isocyanatfunktionellen Prepolymere A) sind durch Umsetzung von Polyisocyanaten A1) mit Polyolen A2) gegebenenfalls unter Zusatz von Katalysatoren sowie Hilfs- und Zusatzstoffen erhältlich.

Als Polyisocyanate A1) können beispielsweise monomere aliphatische oder cycloaliphatische Di- oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexa-methylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-1,8-octandiisocyanat (Nonan-triisocyanat), sowie Alkyl-2,6-diisocyanatohexanoat (Lysindiisocyanat) mit C1-C8-Alkylgruppen eingesetzt werden.

Neben den vorstehend genannten monomeren Polyisocyanaten A1) können auch deren höhermolekulare Folgeprodukte mit Uretdion-, Isocyanurat-, Urethan-, Allophanat-, Biuret-, Iminooxadiazindion- oder Oxadiazintrionstruktur sowie deren Mischungen eingesetzt werden.

Bevorzugt werden Polyisocyanate A1) der vorstehend genannten Art mit ausschließlich aliphatisch oder cycloaliphatisch gebundenen Isocyanatgruppen oder deren Mischungen eingesetzt.

Ebenfalls bevorzugt ist, wenn Polyisocyanate A1) der vorstehenden Art mit einer mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 verwendet werde.

Ganz besonders bevorzugt wird Hexamethylendiisocyanat als Polyisocyanat A1) eingesetzt

Gemäß einer bevorzugten Ausführungsform des erfindungsgemäßen Polyharnstoff-Systems ist vorgesehen, dass die Polyole A2) Polyesterpolyole und/oder Polyester-Polyether-Polyole und/oder Polyetherpolyole sind. Insbesondere bevorzugt sind dabei Polyester-Polyether-Polyole und/oder Polyetherpolyole mit einem Ethylenoxidanteil zwischen 60 und 90 Gew.-%.

Bevorzugt ist auch, wenn die Polyole A2) ein zahlenmittleres Molekulargewicht von 4000 bis 8500 g/mol aufweisen.

Geeignete Polyetheresterpolyole werden entsprechend dem Stand der Technik vorzugsweise durch Polykondensation aus Polycarbonsäuren, Anhydriden von Polycarbonsäuren, sowie Estern von Polycarbonsäuren mit leichtflüchtigen Alkoholen, bevorzugt C1 bis C6 Monoolen, wie Methanol, Ethanol, Propanol oder Butanol, mit molar überschüssigem, niedermolekularem und/oder höhermolekularem Polyol hergestellt; wobei als Polyol ethergruppenhaltige Polyole gegebenenfalls in Mischungen mit anderen ethergruppenfreien Polyolen eingesetzt werden.

Selbstverständlich können zur Polyetherestersynthese auch Gemische der höhermolekularen und der niedermolekularen Polyole verwendet werden.

Solche molar überschüssigen niedermolekularen Polyole sind Polyole mit Molmassen von 62 bis 299 Dalton, mit 2 bis 12 C-Atomen und Hydroxylfunktionalitäten von mindestens 2, die weiterhin verzweigt oder unverzweigt sein können und deren Hydroxylgruppen primär oder sekundär sind. Diese niedermolekularen Polyole können auch Ethergruppen aufweisen. Typische Vertreter sind Ethylenglykol, Propandiol-1,2, Propandiol-1,3, Butandiol-1,4, Butandiol-2,3, 2-Methyl-propandiol-1,3, Pentandiol-1,5, Hexandiol-1,6, 3-Methyl-pentandiol-1,5, 1,8-Octandiol, 1,10-Decandiol, 1,12-Dodecandiol, Cyclohexandiol, Diethylenglykol, Triethylenglykol und höhere Homologe, Dipropylenglykol, Tripropylenglykol und höhere Homologe, Glycerin, 1,1,1-Trimethylolpropan, sowie Oligo-tetrahydrofurane mit Hydroxylendgruppen. Selbstverständlich können innerhalb dieser Gruppe auch Gemische verwendet werden.

Molar überschüssige höhermolekulare Polyole sind Polyole mit Molmassen von 300 bis 3000 Dalton, die durch ringöffenende Polymerisation von Epoxiden, bevorzugt Ethylen- und/oder Propylenoxid, sowie durch säurekatalysierte, ringöffnende Polymerisation von Tetrahydrofuran, erhalten werden können. Zur ringöffnenden Polymerisation von Epoxiden können entweder Alkalihydroxide oder Doppelmetallcyanidkatalysatoren verwendet werden.

Als Starter für ringöffnende Epoxidpolymerisationen können alle mindestens bifunktionellen Moleküle aus der Gruppe der Amine und der o.g. niedermolekularen Polyole verwendet werden. Typische Vertreter sind 1,1,1-Trimethylolpropan, Glycerin, o-TDA, Ethylendiamin, Propylenglykol-1,2, etc. sowie Wasser, einschließlich deren Gemische. Selbstverständlich können innerhalb der Gruppe der überschüssigen höhermolekularen Polyole auch Gemische verwendet werden.

Der Aufbau der höhermolekularen Polyole, soweit es sich um Hydroxylgruppen terminierte Polyalkylenoxide aus Ethylen- und/oder Propylenoxid handelt, kann statistisch oder blockweise erfolgen, wobei auch Mischblöcke enthalten sein können.

Polycarbonsäuren sind sowohl aliphatische als auch aromatische Carbonsäuren, die sowohl cyclisch, linear, verzweigt oder unverzweigt sein können und die zwischen 4 und 24 C-Atome aufweisen können.

Beispiele sind Bernsteinsäure, Glutarsäure, Adipinsäure, Azelainsäure, Sebacinsäure, 1,10-Decandicarbonsäure, 1,12-Dodecandicarbonsäure, Phthalsäure, Terephthalsäure, Isophthalsäure, Trimellitsäure, Pyromellitsäure. Bevorzugt sind Bernsteinsäure, Glutarsäure, Adipinsäure, Sebacinsäure, Milchsäure, Phthalsäure, Terephthalsäure, Isophthalsäure, Trimellitsäure, Pyromellitsäure. Besonders bevorzugt sind Bernsteinsäure, Glutarsäure und Adipinsäure.

Weiterhin umfasst die Gruppe der Polycarbonsäuren auch Hydroxycarbonsäuren, bzw. deren innere Anhydride, wie z.B. Caprolacton, Milchsäure, Hydroxybuttersäure, Ricinolsäure, usw. Mit umfasst sind weiterhin auch Monocarbonsäuren, insbesondere solche, die über mehr als 10 C-Atome verfügen, wie Sojaölfettsäure, Palmölfettsäure und Erdnussölfettsäure, wobei deren Anteil am gesamten, das Polyetheresterpolyol aufbauenden Reaktionsmischung 10 Gew.-% nicht übersteigt und zusätzlich die dadurch einhergehende Minderfunktionalität durch Mitverwendung von mindestens trifunktionellen Polyolen, sei es auf Seiten der niedermolekularen oder der hochmolekularen Polyole ausgeglichen wird.

Die Herstellung der Polyetheresterpolyol erfolgt entsprechend dem Stand der Technik bei erhöhter Temperatur im Bereich von 120 bis 250 °C, zunächst unter Normaldruck, später unter Anlegen von Vakuum von 1 bis 100 mbar, vorzugsweise, aber nicht notwendigerweise unter Verwendung eines Veresterungs- oder Umesterungskatalysators, wobei die Reaktion so weit vervollständigt wird, dass die Säurezahl auf Werte von 0,05 bis 10 mg KOH/g, bevorzugt 0,1 bis 3 mg KOH/g und besonders bevorzugt 0,15 bis 2,5 mg KOH/g absinkt.

Weiterhin kann im Rahmen der Normaldruckphase vor dem Anlegen von Vakuum ein Inertgas verwendet werden. Selbstverständlich können alternativ oder für einzelne Phasen der Veresterung auch flüssige oder gasförmige Schleppmittel zum Einsatz kommen. Beispielsweise kann das Reaktionswasser unter Verwendung von Stickstoff als Trägergas, ebenso ausgetragen werden, wie unter Einsatz eines Azeotropschleppmittels, wie z.B. Benzol, Toluol, Xylol, Dioxan, etc.

Selbstverständlich können auch Abmischungen von Polyetherpolyolen mit Polyesterpolyolen in beliebigen Verhältnissen eingesetzt werden.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Ebenfalls können Hydroxylgruppen aufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art in Frage.

Zur Herstellung des Prepolymers A) kann das Polyisocyanat A1) mit dem Polyol A2) bei einem NCO/OH-Verhältnis von bevorzugt 4:1 bis 12:1, besonders bevorzugt 8:1 umgesetzt und anschließend der Anteil an nicht umgesetzten Polyisocyanates mittels geeigneter Methoden abgetrennt werden. Üblicherweise wird hierfür die Dünnschichtdestillation verwendet, wobei Prepolymere mit Restmonomergehalten von weniger als 1 Gew.-%, bevorzugt weniger als 0,1 Gew.-%, ganz besonders bevorzugt weniger als 0,03 Gew.-% erhalten werden.

Gegebenenfalls können während der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur bei der Herstellung der Prepolymere A) beträgt dabei bevorzugt 20 bis 120 °C und weiter bevorzugt 60 bis 100 °C.

Die hergestellten Prepolymere haben einen nach DIN EN ISO 11909 gemessenen mittleren NCO-Gehalt von 2 bis 10 Gew.-%, bevorzugt 2,5 bis 8 Gew.-%.

Gemäße einer weiteren Ausführungsform des erfindungsgemäßen Polyharnstoff-Systems können die Prepolymere A) eine mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 aufweisen.

Bei den organischen Füllstoffen der Komponente C) kann es sich bevorzugt um hydroxyfunktionelle Verbindungen, insbesondere um Polyetherpolyole mit sich wiederholenden Ethylenoxid Einheiten handelt.

Vorteilhaft ist auch, wenn die Füllstoffe der Komponente C) eine mittlere OH-Funktionalität von 1,5 bis 3, bevorzugt von 1,8 bis 2,2 und besonders bevorzugt von 2 aufweisen.

Beispielsweise können als organische Füllstoffe bei 23 °C flüssige Polyethylenglykole wie PEG 200 bis PEG 600, deren Mono- bzw. Dialkylether wie PEG 500 Dimethylether, flüssige Polyether- und Polyesterpolyole, flüssige Polyester wie z.B. Ultramoll (Lanxess AG, Leverkusen, DE) sowie Glycerin und seine flüssigen Derivate wie z.B. Triacetin (Lanxess AG, Leverkusen, DE) eingesetzt werden.

Die Viskosität der organischen Füllstoffe gemessen nach DIN 53019 bei 23 °C beträgt bevorzugt 50 bis 4000 mPas, besonders bevorzugt 50 bis 2000 mPas.

In einer bevorzugten Ausführungsform des erfindungsgemäßen Polyharnstoff-Systems werden als organische Füllstoffe Polyethylenglykole eingesetzt. Diese haben bevorzugt ein zahlenmittleres Molekulargewicht von 100 bis 1000 g/mol, besonders bevorzugt 200 bis 400 g/mol.

Um das mittlere Equivalentgewicht der insgesamt zur Prepolymervernetzung eingesetzten Verbindungen bezogen auf die NCO-reaktiven Gruppen weiter zu reduzieren, ist es möglich zusätzlich Umsetzungsprodukte der Prepolymere A) mit der aminofunktionellen Verbindung B) und/oder den organischen Füllstoffen C), sofern diese amino- oder hydroxyfunktionell sind, in einer separaten Vorreaktion herzustellen und dann als höhermolekulare Härterkomponente einzusetzen.

Bevorzugt werden bei der Vorverlängerung Verhältnisse von isocyanatreaktiven Gruppen zu Isocyanatgruppen von 50 zu 1 bis 1,5 zu 1, besonders bevorzugt 15 zu 1 bis 4 zu 1 eingestellt.

Vorteil dieser Modifizierung durch Vorverlängerung ist, dass das Equivalentgewicht und das Equivalentvolumen der Härterkomponente in größeren Grenzen modifizierbar ist. Dadurch können zur Applikation kommerziell verfügbare 2-Kammerdosiersysteme eingesetzt werden, um ein Klebesystem zu erhalten, das bei bestehenden Verhältnissen der Kammervolumina in das gewünschte Verhältnis von NCO-reaktiven Gruppen zu NCO-Gruppen eingestellt werden kann.

Gemäß einer weiteren bevorzugten Ausführungsform des erfindungsgemäßen Polyharnstoff-Systems ist vorgesehen, dass die Komponente E) ein tertiäres Amin der allgemeinen Formel (III) 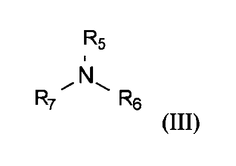 enthält, in der
- R₅, R₆, R₇: unabhängig voneinander Alkyl- oder Heteroalkyreste mit Heteroatomen in der Alkylkette oder an deren Ende sein können, oder R₇ und R₈ gemeinsam mit dem sie tragenden Stickstoffatom einen aliphatischen, ungesättigten oder aromatischen Heterozyklus bilden können, der gegebenenfalls weitere Heteroatome enthalten kann.

Diese Polyharnstoff-Systeme zeichnen sich durch eine besonders schnelle Aushärtung aus.

Bei den in Komponente E) verwendeten Verbindungen kann es sich ganz besonders bevorzugt um tertiäre Amin ausgewählt aus der Gruppe Triethanolamin, Tetrakis (2-hydroxyethyl) ethylendiamin, N,N-Dimethyl-2-(4-methylpiperazin-1-yl)ethanamin, 2-{[2-(Dimethylamino)ethyl](methyl)amino}-ethanol, 3,3',3"-(1,3,5-Triazinan-1,3,5-triyl)tris(N,N-dimethyl-propan-1-amin) handeln.

Ganz besonders hohe Aushärtungsgeschwindigkeiten können auch erzielt werden, wenn die Komponente E) 0,2 bis 2,0 Gew.-% Wasser und/oder 0,1 bis 1,0 Gew.-% des tertiäres Amin enthält.

Selbstverständlich können in die Polyharnstoff-Systeme auch pharmakologisch aktive Wirkstoffe wie Analgetika mit und ohne antiinflammatorische Wirkung, Antiphlogistika, antimikrobiell wirksame Substanzen, Antimykotika, antiparasitär wirkende Stoffe umfassen.

Das erfindungsgemäße Polyharnstoff-System ist besonders zum Verschließen, Verbinden, Verkleben oder Abdecken von Zellgewebe und insbesondere zur Stillung des Austritts von Blut oder Gewebeflüssigkeiten oder dem Verschluss von Leckagen in Zellgewebe geeignet. Ganz besonders bevorzugt kann es zur Verwendung oder zur Herstellung eines Mittels zum Verschließen, Verbinden, Verkleben oder Abdecken von menschliches oder tierisches Zellgewebe verwendet werden. Mit seiner Hilfe können schnell aushärtende, stark am Gewebe anhaftende, transparente, flexible und biokompatible Klebnähte hergestellt werden.

Noch ein weiterer Gegenstand der Erfindung ist Dosiersystem mit zwei Kammern für ein erfindungsgemäßes Polyharnstoff-System, bei dem in der einen Kammer die Komponente A) und in der anderen Kammer die Komponenten B) und gegebenenfalls die Komponenten C), D) und. E) des Polyharnstoff-Systems enthalten sind. Ein derartiges Dosiersystem eignet sich insbesondere dafür das Polyharnstoff-System als Kleber auf Gewebe zu applizieren.

### Beispiele:

### Methoden:

Molekulargewicht: Die Molekulargewichte wurden mittels Gelpermeationschromatographie (GPC) wie folgt bestimmt: Die Kalibrierung erfolgt mit Polystyrol-Standards mit Molekulargewichten von Mp 1.000.000 bis 162. Als Eluent wurde Tetrahydrofuran p.A. verwendet. Die folgenden Parameter wurden bei der Doppelmessung eingehalten: Entgasung: Online - Degasser; Durchfluß: 1 ml/Min; Analysenzeit: 45 Minuten; Detektoren: Refraktometer und UV-Detektor; Injektionsvolumen: 100 µl - 200 µl. Die Berechnung der Molmassenmittelwerte Mw; Mn und Mp sowie der Polydispersität Mw/Mn erfolgte softwaregestützt. Basislinienpunkte und Auswertegrenzen wurden entsprechend der DIN 55672 Teil 1 festgelegt.

| | |
|---|---|
| NCO-Gehalt: | wenn nicht ausdrücklich anders erwähnt, volumetrisch gemäß DIN-EN ISO 11909 bestimmt. |
| Viskosität: | Wurde nach ISO 3219 bei 23°C bestimmt. |
| Restmonomergehalt: | erfolgte nach DIN ISO 17025 |
| NMR: | Wurde mit einem Bruker DRX 700 Gerät bestimmt. |

### Substanzen:

HDI: Hexamethylendiisocyanat (Bayer MaterialScience AG)
Polyethylenglykol 600 (Aldrich)
Polyethylenglykol 400 (Aldrich)
Polyethylenglykol 200 (Aldrich)

### Synthese von Triethyl-4-oxo-3-oxa-7,11,16-triazaoctadecan-6,17,18-tricarboxylat (Spermidinaspartat)(1)

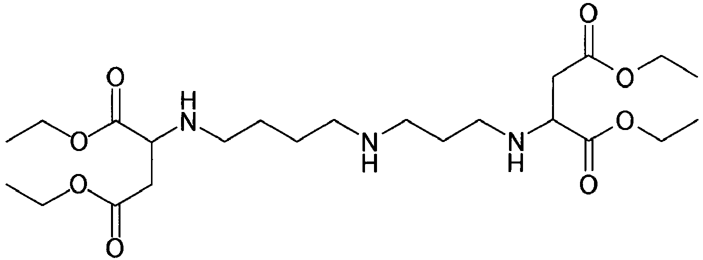

Zu einer Lösung von 5 g (34,5 mmol) Spermidin in 5 ml THF wurden 11,9 g (69 mmol) Maleinsäurediethylester gegeben. Die Reaktionsmischung wurde 3 Tage bei 60 °C gerührt. Nach Entfernen des Lösemittels im Vakuum wurde das Produkt quantitativ als gelbe Flüssigkeit erhalten.
¹H-NMR (CDCl₃, 700 MHz): δ = 1.27 (t, 6H), 1,29 (t, 6H), 1.49 (br, 3NH), 1.62 (m, 6H) 2,62 (m, 12H), 3.60 (t, 2H), 4.13 (q, 4H), 4.2 (q, 4H).
¹³C-NMR (CDCl₃, 700 MHz): 13.7, 27.3, 27.4, 29.8, 37.6, 46.1, 47.4, 47.7, 49.3, 57.3, 59.9, 60.1, 170.9, 173.1.

### Synthese von Triethyl-4-oxo-3-oxa-7,14,21-triazatricosan-6,22,23-tricarboxylat (2)

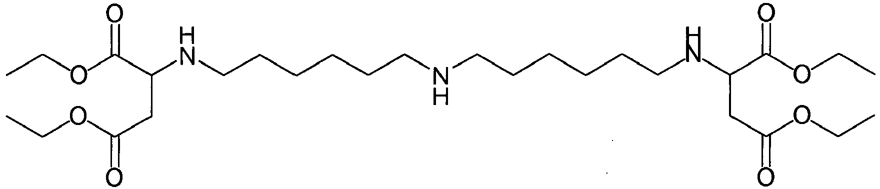

Analog zu (1) wurden aus 154 g (0,72 Mol) Bis(hexamethylen)-triamin und 246 g (1,42 Mol) Maleinsäurediethylester 400 g des Produktes als gelbe Flüssigkeit erhalten.
¹H-NMR (CDCl₃, 700 MHz): δ = 1.27 (t, 6H), 1,29 (t, 6H), 1.34 (m, 8H), 1.49 (br, 3NH), 2,59 (m, 12H), 3.70 (t, 2H), 4.11 (q, 4H), 4.2 (q, 4H).
¹³C-NMR (CDCl₃, 700 MHz): 14.1, 26.8, 27.1, 30.0, 30.1, 38.1, 47.9, 50.1, 57.8, 60.3, 60.8, 170.7, 172.7.

### Triethyl-4-oxo-3-oxa-7,11,15-triazaheptadecan-6,16,17-tricarboxylat (3)

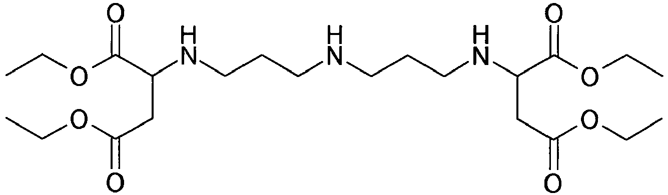

Analog zu (1) wurden aus 3,9 g (0,03 Mol) Bis(3-Aminopropyl)amin und 10,33 g (0,06 Mol) Maleinsäurediethylester 14,23 g des Produktes als gelbe Flüssigkeit erhalten.
¹H-NMR (CDCl₃, 700 MHz): δ = 1.26 (t, 6H), 1,27 (t, 6H), 1.49 (br, 3NH), 1.62 (m, 4H), 2,61 (m, 12H), 3.60 (t, 2H), 4.15 (q, 4H), 4.19 (q, 4H).
¹³C-NMR (CDCl₃, 700 MHz): 14.2, 14.4, 27.9, 30.3, 38.1, 46.5, 48.1, 49.9, 57.8, 60.4, 61.0, 170.8, 173.2.

### Synthese von Prepolymer A

212,5 g (1,8 mol) Bersteinsäure wurden mit 1591,5 g Polyethylenglykol 600 (2,6 mol) unter Rühren auf 235 °C erhitzt. Dabei wurde über 8,5 h das entstehende Wasser abdestilliert. Anschließend wurden 100 ppm Zinn(II)chlorid hinzugegeben und für weitere 9 h unter Vakuum (15 mbar) am Wasserabscheider auf 235°C erhitzt.

672 g HDI (4 mol) wurden mit 0,1 Gew% Benzoylchlorid vorgelegt und auf 80 °C erhitzt. Anschließend wurden unter Rühren 788 g des zuvor hergestellten Polyesters über 1h zudosiert und bis zum Erreichen eines konstanten NCO-Gehalts bei 80 °C weiter gerührt. Das überschüssige HDI wurde bei 140°C und 0,13 mbar mittels Dünnschichtverdampfer entfernt. Das erhaltene Prepolymer hatte einen NCO-Gehalt von 3,5 % und eine Viskosität von 4700 mPas/23°C. Der Restmonomerengehalt betrug < 0,03 % HDI.

### Synthese von Prepolymer B

Analog zu Prepolymer A wurde aus 263 g (1,8 mol) Adipinsäure und 1591,5 g Polyethylenglykol 600 (2,6 mol) der entsprechende Polyester hergestellt und mit HDI zum Prepolymer umgesetzt. Das erhaltene Prepolymer hatte einen NCO-Gehalt von 5,93 % und eine Viskosität von 1450 mPas/23°C. Der Restmonomerengehalt betrug < 0,03 % HDI.

### Vergleichsbeispiel trifunktioneller Ester: Synthese von Prepolymer C

Analog zu Prepolymer A wurden 236,2 g (2 mol) Bernsteinsäure, 800 g Polyethylenglykol 400 (2 mol) und 29,84 g Glycerin (0,324 mol) zu dem entsprechenden Ester umgesetzt. Das daraus erhaltene HDI-Prepolymer hatte einen NCO-Gehalt von 3,2 % und eine Viskosität von 50.100 mPas/23°C. Der Restmonomerengehalt betrug < 0,03 % HDI.

### Vergleichsbeispiel trifunktioneller Ester: Synthese von Prepolymer D

Analog zu Prepolymer A wurden 141,7 g (1,2 mol) Bernsteinsäure, 720 g Polyethylenglykol 200 (1,2 mol) und 25,42 g Glycerin (0,276 mol) zu dem entsprechenden Ester umgesetzt. Das daraus erhaltene HDI-Prepolymer hatte einen NCO-Gehalt von 2,7 % und eine Viskosität von 42.500 mPas/23°C. Der Restmonomerengehalt betrug < 0,03 % HDI.

### Herstellung des Gewebeklebers

4 g des jeweiligen Prepolymers wurden mit einer equivalenten Menge der hergestellten aminofunktionellen Verbindung 3 in einem Becher gut verrührt. Das Polyharnstoff-System wurde unmittelbar danach auf das zu klebende Muskelgewebe als dünne Schicht aufgetragen. Als Verarbeitungszeit wurde dabei die Zeit bestimmt, innerhalb der das Polyharnstoff-System noch eine so niedrige Viskosität besaß, dass es problemlos auf das Gewebe aufgetragen werden könnte.

Die Zeit, nach der das Polyharnstoff-System nicht mehr klebrig war (tack free time) wurde durch Haftversuche mit einem Glasstab gemessen. Dazu wurde der Glasstab mit der Schicht aus dem Polyharnstoff-System in Kontakt gebracht. Blieb dieser nicht mehr haften, wurde die System als tack free angesehen. Zusätzlich wurde die Klebekraft bestimmt, indem zwei Stücke Muskelgewebe (1= 4 cm, h = 0.3 cm, b = 1 cm) an den Enden 1 cm weit mit dem Polyharnstoff-System bestrichen und überlappend geklebt wurden. Die Klebkraft des Polyharnstoff-Systems wurde jeweils durch Zug überprüft.

| | Härter | Verarbeitungszeit | Tack free time | Klebekraft |
|---|---|---|---|---|
| Prepolymer A | **1** | 1 min 40 s | 3 min | ++ |
| Prepolymer A | **2** | 1 min | 2 min | ++ |
| Prepolymer A | **3** | 1 min 50s | 3 min | ++ |
| Prepolymer B | **1** | 1 min | 2 min 30 s | + |
| Prepolymer B | **2** | 1 min | 2 min | + |
| Prepolymer B | **3** | 1 min | 2 min 30 s | + |
| Prepolymer B (Vergleichsbeispiel 1) | **6** | 8 min | > 30 min | + |

| | | | | |
|---|---|---|---|---|
| ++: sehr gut; +: gut | | | | |

### Vergleichsbeispiel: Difunktionelles Prepolymer + difunktioneller Härter

Anstelle des beschriebenen Härters 2 wurde das in EP 2 145 634 beschriebene Tetraethyl-2,2'-[(2-methylpentan-1,5-diyl)diimino]dibutandioat (6) eingesetzt. Die Verarbeitungszeit mit Prepolymer B lag bei 8 min. Der Klebstoff war auch nach 30 min nicht tack free.

### Messung der Zytotoxizität eines mit (2) hergestellten Klebstoffs

Das Prepolymer A wurde mit einer äquivalenten Menge 2 ausgehärtet. Die Zytotoxizität wurde gemäß ISO 10993-5:2009 mit L929 Zellen gemessen. Es hat keine Herabsetzung der Zellviabilität stattgefunden. Das System ist damit nicht als zytotoxisch einzustufen.

## Patentansprüche

1. Polyhrnstoff-System zum Verschließen, Verbinden, Verkleben oder Abdecken von Zellgewebe, umfassend
als Komponente A) isocyanatfunktionelle Prepolymere erhältlich durch Umsetzung von aliphatischen Isocyanaten A1) mit
Polyolen A2), die insbesondere ein zahlenmittleres Molekulargewicht von ≥ 400 g/mol und eine mittlere OH-Funktionalität von 2 bis 6 aufweisen können,
als Komponente B) aminofunktionelle Asparaginsäureester der allgemeinen Formel (I) 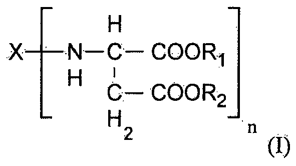 in der
X ein organischer Rest enthaltend eine sekundäre Aminofunktion ist,
R₁, R₂ gleiche oder verschiedene organische Reste sind, die keinen Zerewitinoffaktiven Wasserstoff aufweisen und
n eine ganze Zahl von mindestens 2 ist.

2. Polyharnstoff-System nach Anspruch 1, **dadurch gekennzeichnet, dass** X ein Rest der Formel (II) 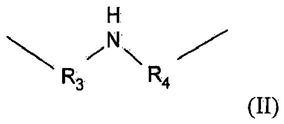 ist, in der
R₃, R₄ jeweils unabhängig voneinander ein organischer Rest sind, der keinen Zerewitinoff-aktiven Wasserstoff aufweist.

3. Polyharnstoff-System nach Anspruch 2, **dadurch gekennzeichnet, dass** R₃, R₄ jeweils unabhängig voneinander oder gleichzeitig ein linearer oder verzweigter, gegebenenfalls auch in der Kette mit Heteroatomen substituierter gesättigter organischer Rest, insbesondere ein linearer oder verzweigter, gesättigter, aliphatischer C1 bis C10, bevorzugt C2 bis C8 und besonders bevorzugt C2 bis C6 Kohlenwasserstoffrest sind.

4. Polyharnstoff-System nach einem der Ansprüche 1 bis 3, **dadurch gekennzeichnet, dass** die Reste R₁, R₂ jeweils unabhängig voneinander lineare oder verzweigte C1 bis C 10, bevorzugt C1 bis C8, besonders bevorzugt C2 bis C6, ganz besonders bevorzugt C2 bis C4 organische Reste und insbesondere aliphatische Kohlenwasserstoffreste sind

5. Polyharnstoff-System nach einem der Ansprüche 1 bis 4, **dadurch gekennzeichnet, dass** die Polyole A2) Polyesterpolyole und/oder Polyester-Polyether-Polyole und/oder Polyetherpolyole und insbesondere Polyester-Polyether-Polyole und/oder Polyetherpolyole mit einem Ethylenoxidanteil zwischen 60 und 90 Gew.-%, enthalten.

6. Polyharnstoff-System nach einem der Ansprüche 1 bis 5, **dadurch gekennzeichnet, dass** die Polyole A2) ein zahlenmittleres Molekulargewicht von 4000 bis 8500 g/mol aufweisen.

7. Polyharnstoff-System nach einem der Ansprüche 1 bis 6, **dadurch gekennzeichnet, dass** es als Komponente C) organische Füllstoffe umfasst, die insbesondere eine nach DIN 53019 gemessenen Viskosität bei 23 °C im Bereich von 10 bis 20.000 mPas aufweisen können.

8. Polyharnstoff-System nach Anspruch 7, **dadurch gekennzeichnet, dass** die organischen Füllstoffe hydroxyfunktionelle Verbindungen sind.

9. Polyharnstoff-System nach Anspruch 8, **dadurch gekennzeichnet, dass** die hydroxyfunktionellen Verbindungen Polyetherpolyole sind.

10. Polyharnstoff-System nach Anspruch 8 oder 9, **dadurch gekennzeichnet, dass** die die hydroxyfunktionellen Verbindungen eine mittlere OH-Funktionalität von 1,5 bis 3, bevorzugt von 1,8 bis 2,2 und insbesondere bevorzugt von 2 aufweisen.

11. Polyharnstoff-System nach einem der Ansprüche 1 bis 10, **dadurch gekennzeichnet, dass** es als Komponente D) Wasser und/oder ein tertiäres Amin enthält, wobei das tertiäre Amin insbesondere aus der Gruppe Triethanolamin, Tetrakis (2-hydroxyethyl) ethylendiamin, N,N-Dimethyl-2-(4-methylpiperazin-1-yl)ethanamin, 2-{[2-(Dimethylamino)ethyl](methyl)amino}ethanol, 3,3',3"-(1,3,5-Triazinan-1,3,5-triyl)tris(N,N-dimethyl-propan-1-amin)ausgewählt ist.

12. Polyharnstoff-System nach Anspruch 11, **dadurch gekennzeichnet, dass** es 0,2 bis 2,0 Gew.-% Wasser und/oder 0,1 bis 1,0 Gew.-% des tertiären Amins enthält.

13. Polyharnstoff-System nach einem der Ansprüche 1 bis 12, **dadurch gekennzeichnet, dass** es als Komponente E) pharmakologisch aktive Verbindungen, insbesondere Analgetika mit oder ohne antiinflammatorische Wirkung, Antiphlogistika, antimikrobiell wirksame Substanzen oder Antimykotika umfasst.

14. Polyharnstoff-System nach einem der Ansprüche 1 bis 13 zur Stillung des Austritts von Blut oder Gewebeflüssigkeiten oder dem Verschluss von Leckagen in Zellgewebe.

15. Dosiersystem mit zwei Kammern für ein Polyharnstoff-System nach einem der Ansprüche 1 bis 14, **dadurch gekennzeichnet, dass** in der einen Kammer die Komponente A) und in der anderen Kammer die Komponenten B) sowie gegebenenfalls die Komponenten C), D) und E) des Polyharnstoff-Systems enthalten sind.

## Claims

1. Polyurea system for sealing, bonding, gluing or covering of cell tissue,
comprising
as component A) isocyanate-functional prepolymers that may be obtained by reacting aliphatic isocyanates A1) with
polyols A2), in particular those having a number-average molecular weight of ≧400 g/mol and an average OH functionality between 2 to 6, and
as component B) amino-functional aspartic acid esters of general formula (I) 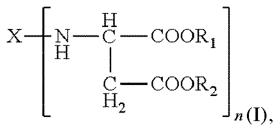 wherein
X represents an organic group containing a secondary amino functionality,
R₁, R₂ represent identical or different organic groups which do not contain a Zerewitinoff-active hydrogen and
n is an integer of at least 2.

2. Polyurea system according to claim 1, **characterized in that** X represents a group of formula (II) 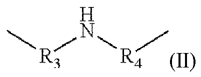 wherein
R₃, R₄ independently of one another represent an organic group that does not contain a Zerewitinoff active hydrogen.

3. Polyurea system according to claim 2, **characterized in that** R₃, R₄ independantly of one another or at the same time represent a straight-chained or branched organic group, optionally substituted in the chain by heteroatoms, in particular a straight-chained or branched saturated aliphatic C1 to C10, preferably C2 to C8 and particularly preferably C2 to C6 hydrocarbon group.

4. Polyurea system according to any one of claims 1 to 3, **characterized in that** R₁, R₂ independently of one another present straight-chained or branched C1 to C10, preferably C1 to C8, particularly preferably C2 to C6, even more preferably C2 to C4 organic groups and in particular aliphatic hydrocarbon groups.

5. Polyurea system according to any one of claims 1 to 4, **characterized in that** the polyols A2) contain polyester polyols and/or polyester-polyether polyols and/or polyether polyols and in particular polyester-polyether polyols and/or polyether polyols having an ethylene oxide content between 60 and 90 wt -%.

6. Polyurea system according to any one of claims 1 to 5, **characterized in that** the polyols A2) have a number average molecular weight from 4,000 to 8,500 g/mol.

7. Polyurea system according to any one of claims 1 to 6, **characterized in that** said system contains as component C) organic fillers having in particular a measured viscosity at 23°C in the range between 10 to 20,000 mPas when measured in accordance with DIN 53 019.

8. Polyurea system according to claim 7, **characterized in that** the organic fillers are hydroxy functional compounds.

9. Polyurea system according to claim 8, **characterized in that** the hydroxy-functional compounds are polyether polyols.

10. Polyurea system according to any one of claims 8 or 9, **characterized in that** the hydroxy-functional compounds have an average OH functionality between 1.5 to 3, preferably between 1.8 to 2.2 and particularly preferably of 2.

11. Polyurea system according to any one of claims 1 to 10, **characterized in that** as component D) said system contains water and/or a tertiary amine, wherein the tertiary amine is in particular selected from the group consisting of triethanolamine, tetrakis (2-hydroxyethyl) ethylenediamine, N, N-dimethyl-2-(4-methylpiperazin-1-yl)ethanamine, 2-{[2-(dimethylamino)ethyl] (methyl)-amino }ethanol, 3,3',3"-(1,3,5-triazinan-1,3,5-triyl)tris(NN-dimethyl-propan-1-amine).

12. Polyurea system according to claim 11, **characterized in that** said system contains 0.2 to 2.0 wt -% water and/or 0.1 to 1.0 wt % of the tertiary amine.

13. Polyurea system according to any one of claims 1 to 12, **characterized in that** said system comprises as component E) pharmacologically active compounds, in particular analgesics with or without anti-inflammatory action, anti-phlogistics, anti-microbially effective substances or anti-mycotics.

14. Polyurea system according to any one of claims 1 to 13 for stemming the flow of blood or tissue fluids, or the sealing of leaks in cellular tissue.

15. Dosing system comprised of two chambers for a polyurea system according to any one of claims 1 to 14, **characterized in that** one chamber contains component A) and the other chamber contains component B) and, optionally C), D) and E) of the poly urea system.

## Revendications

1. Système de polyurée destiné à fermer, lier, agglutiner ou recouvrir des tissus cellulaires comprenant
comme composant A) des prépolymères fonctionnels isocyanate pouvant être obtenus par transformation d'isocyanates aliphatiques A1) avec des polyols A2), lesquels peuvent présenter en particulier un poids moléculaire moyen en nombre supérieur ou égal à 400 g/mol et une fonctionnalité OH moyenne de 2 à 6,
comme composant B) des esters d'acide aspartique aminofonctionnels de formule générale (I) 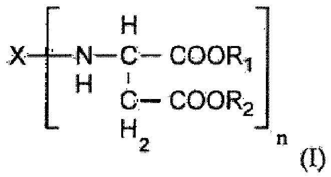 où
X est un groupe fonctionnel organique contenant une fonction amino secondaire,
R₁ et R₂ sont des groupes fonctionnels organiques identiques ou différents ne présentant aucun atome d'hydrogène actif de Zerewitinoff, et
n est un nombre entier équivalant à au moins 2.

2. Système de polyurée selon la revendication 1, **caractérisé en ce que** X est un groupe fonctionnel de formule (II) 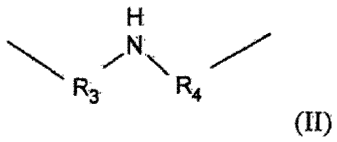 où
R₃ et R₄ sont chacun, indépendamment l'un de l'autre, des groupes fonctionnels organiques ne présentant aucun atome d'hydrogène actif de Zerewitinoff.

3. Système de polyurée selon la revendication 2, **caractérisé en ce que** R₃ et R₄ sont chacun, indépendamment l'un de l'autre ou simultanément, des groupes fonctionnels organiques linéaires ou ramifiés, saturés, le cas échéant substitués dans la chaîne par des hétéroatomes, en particulier des groupes hydrocarbures linéaires ou ramifiés, saturés, aliphatiques en C1 à C10, de préférence en C2 à C8 et de façon particulièrement préférée en C2 à C6.

4. Système de polyurée selon l'une des revendications 1 à 3, **caractérisé en ce que** les groupes fonctionnels R₁ et R₂ sont chacun, indépendamment l'un de l'autre, des groupes fonctionnels organiques linéaires ou ramifiés en C1 à C10, de préférence en C1 à C8, plus préférablement en C2 à C6, de façon tout particulièrement préférée en C2 à C4, et particulièrement des groupes fonctionnels hydrocarbures aliphatiques.

5. Système de polyurée selon l'une des revendications 1 à 4, **caractérisé en ce que** les polyols A2) contiennent des polyesterpolyols et/ou des polyester-polyétherpolyols et/ou des polyétherpolyols et tout particulièrement des polyester-polyéther-polyols et/ou des polyétherpolyols présentant une proportion de 60 à 90 % en poids d'oxyde d'éthylène.

6. Système de polyurée selon l'une des revendications 1 à 5, **caractérisé en ce que** les polyols A2) présentent un poids moléculaire moyen en nombre de 4000 à 8500 g/mol.

7. Système de polyurée selon l'une des revendications 1 à 6, **caractérisé en ce qu'**il comprend comme composant C) des matières de charge organiques pouvant notamment présenter une viscosité mesurée à 23 °C selon DIN 53019 comprise entre 10 et 20 000 mPas.

8. Système de polyurée selon la revendication 7, **caractérisé en ce que** les matières de charge organiques sont des composés hydroxyfonctionnels.

9. Système de polyurée selon la revendication 8, **caractérisé en ce que** les composés hydroxyfonctionnels sont des polyétherpolyols.

10. Système de polyurée selon la revendication 8 ou 9, **caractérisé en ce que** les composés hydroxyfonctionnels présentent une fonctionnalité OH moyenne de 1,5 à 3, de préférence 1,8 à 2,2 et particulièrement préférablement de 2.

11. Système de polyurée selon l'une des revendications 1 à 10, **caractérisé en ce qu'**il contient comme composant D) de l'eau et/ou une amine tertiaire, l'amine tertiaire étant notamment sélectionnée dans le groupe triéthanolamine, tetrakis (2-hydroxyéthyl)éthylènediamine, N,N-diméthyl-2-(4-méthylpipérazin-1-yl)éthanamine, 2-{[2-(diméthylamino)éthyl](méthyl)amino}éthanol, 3,3',3"-(1,3,5-triazinan-1,3,5-triyl)tris(N,N-diméthyl-propan-1-amine).

12. Système de polyurée selon la revendication 11, **caractérisé en ce qu'**il contient 0,2 à 2,0 % en poids d'eau et/ou 0,1 à 1,0 % en poids d'amine tertiaire.

13. Système de polyurée selon l'une des revendications 1 à 12, **caractérisé en ce qu'**il comprend comme composant E) des composés pharmacologiquement actifs, notamment des analgésiques avec ou sans effet anti-inflammatoire, des anti-inflammatoires, des substances antimicrobiennes actives ou des antifongiques.

14. Système de polyurée selon l'une des revendications 1 à 13 destiné à stopper une hémorragie ou l'écoulement de fluides tissulaires ou à sceller des fuites dans les tissus cellulaires.

15. Système de dosage à deux chambres pour un système de polyurée selon l'une des revendications 1 à 14, **caractérisé en ce que** le composant A) est contenu dans ladite première chambre et les composants B) sont contenus dans ladite seconde chambre, ainsi que, le cas échéant, les composants C), D) et E) du système de polyurée.
